(19) Europäisches Patentamt
European Patent Office
Office européen des brevets

(11) **EP 4 311 493 A1**

(12) **EUROPEAN PATENT APPLICATION**

(43) Date of publication:
**31.01.2024 Bulletin 2024/05**

(21) Application number: **22187238.5**

(22) Date of filing: **27.07.2022**

(51) International Patent Classification (IPC):
***A61B 6/00*** *(2024.01)*

(52) Cooperative Patent Classification (CPC):
**A61B 6/4241; A61B 6/481; A61B 6/482;**
**A61B 6/504; A61B 6/5205; A61B 6/5217;**
A61B 6/4441

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HAASE, Hans Christian**
**Eindhoven (NL)**

• **GRASS, Michael**
**Eindhoven (NL)**
• **NICKISCH, Hannes**
**5656AG Eindhoven (NL)**
• **SCHMITT, Holger**
**5656AG Eindhoven (NL)**
• **LANGZAM, Eran**
**Eindhoven (NL)**

(74) Representative: **Philips Intellectual Property &**
**Standards**
**High Tech Campus 52**
**5656 AG Eindhoven (NL)**

(54) **SPECTRAL X-RAY PROJECTION DATA**

(57) A computer-implemented method of providing projection data includes: receiving spectral computed tomography data (110), receiving spectral X-ray projection data (130) that is generated using a set value of one or more imaging parameters (140), and extracting material projection data (150) from the spectral X-ray projection data (130). The extraction of the material projection data (150) from the spectral X-ray projection data (130), or the setting of the value of the one or more imaging parameters (140), is performed using the received spectral CT data (110).

FIG. 3

EP 4 311 493 A1

**Description**

TECHNICAL FIELD

The present disclosure relates to providing material projection data from spectral X-ray projection data. A computer-implemented method, a computer program product, and a system, are disclosed.

BACKGROUND

Spectral CT imaging is increasingly finding application in settings in which conventional CT imaging has traditionally been used. For instance, spectral CT imaging is currently used in the evaluation of aortic disease, the evaluation of coronary artery lumens, the assessment of myocardial perfusion, and the detection of pulmonary embolisms. Spectral CT data includes data from multiple different energy intervals, and the processing of such data permits a distinction between materials that have similar X-ray attenuation values when measured within a single energy interval, and which would be indistinguishable in conventional CT data.

Over time, it is also expected that spectral projection imaging will find application in settings in which conventional X-ray projection imaging systems have been used. For example, it is expected that spectral projection imaging will find application in settings such as the cath lab, and used in investigations for coronary artery disease "CAD", and also in treatment procedures such as percutaneous coronary interventions "PCI". Such investigations typically involve an assessment of blood flow by means of parameters such as the FFR, and these assessments are increasingly being performed using angiographic data, obviating the need for invasive measurement devices such as a pressure-wire.

A spectral X-ray projection imaging system acquires spectral X-ray projection data with its source-detector arrangement in a static position with respect to a region of interest. This contrasts with a spectral CT imaging system, and in which spectral CT data is acquired whilst rotating, or stepping a source-detector arrangement around a region of interest. As a consequence, spectral CT data has sufficient information for the reconstruction of volumetric images of a region of interest, whereas spectral X-ray projection data is conventionally used only to generate 2D images.

The use of spectral X-ray projection data has several advantages over spectral CT data, however. For instance, spectral X-ray projection data can be generated by spectral X-ray projection imaging systems, and these typically have improved versatility, lower complexity, lower cost, and they can often generate images with a higher image resolution and lower X-ray dose than spectral CT imaging systems. These benefits can be exploited in settings such as the cath lab, and in which it is important to maximize access to a patient when performing clinical investigations and treatment procedures.

However, since spectral X-ray projection data represents a region of interest from a single perspective, features in a region of interest overlap one another in spectral X-ray projection data. This complicates the extraction of material data for the features in the region of interest. Consequently, in order to exploit the benefits of improved distinction between the materials of these features that is offered by spectral X-ray projection imaging systems, there is a need for improvements in the provision of projection data by spectral X-ray projection imaging systems.

SUMMARY

According to one aspect of the present disclosure, a computer-implemented method of providing projection data is provided. The method includes:

receiving spectral computed tomography, CT, data representing a region of interest, the spectral CT data defining X-ray attenuation within the region of interest in a plurality of different energy intervals;
receiving spectral X-ray projection data representing the region of interest, the spectral X-ray projection data defining X-ray attenuation within the region of interest in the plurality of energy intervals, and being generated using a set value of one or more imaging parameters; and
extracting, from the spectral X-ray projection data, material projection data representing X-ray attenuation of one or more materials in the region of interest, to provide the projection data; and
wherein the extracting material projection data from the spectral X-ray projection data, or the setting of the value of the one or more imaging parameters, is performed using the received spectral CT data.

In the above method, the extraction of the material projection data from the spectral X-ray projection data, or the setting of the value of the one or more imaging parameters, is performed using spectral CT data for the region of interest. The spectral CT data represents the volumetric extent of features in the region of interest, and consequently provides more accurate spatial information on the spectral attenuation by the features. By extracting the material projection data using the spectral CT data, spatially-accurate spectral information for the features is used to extract the material projection data, and consequently the resulting material projection data for the features is more specific to the intended material(s). By setting the value(s) of imaging parameters, such as e.g. a dose, a spectrum of X-rays, and an orientation of the imaging system used to generate the spectral X-ray projection data, using the spectral CT data, it can be ensured that the spectral X-ray projection data includes sufficient information to accurately extract the material projection data for a material. Consequently, both uses of the spectral CT data provide that the extracted material projection data is more specific and more accurate to the intended material(s). This enables a physician to make a more accurate diagnosis of the region of interest from the extracted material projection data.

Further aspects, features, and advantages of the present disclosure will become apparent from the following description of examples, which is made with reference to the accompanying drawings.

BRIEF DESCRIPTION OF THE DRAWINGS

Fig. 1 is a flowchart illustrating an example of a computer-implemented method of providing projection data, in accordance with some aspects of the present disclosure.

Fig. 2 is a schematic diagram illustrating an example of a system 200 for providing projection data, in accordance with some aspects of the present disclosure.

Fig. 3 is a schematic diagram illustrating an example of a computer-implemented method of providing projection data, in accordance with some aspects of the present disclosure.

Fig. 4 is a schematic diagram illustrating a heart, including an example of a vessel 180, in accordance with some aspects of the present disclosure.

Fig. 5 is a graph illustrating the dependence of the mass attenuation coefficient with X-ray energy for two example materials, iodine and water.

Fig. 6 is a schematic diagram illustrating an example of the operation of projecting spectral CT data 110 to provide simulated spectral X-ray projection data $110_P{}'$, in accordance with some aspects of the present disclosure.

Fig. 7 is a schematic diagram illustrating an example of a first orientation parameter $\alpha$ that defines an orientation of an imaging system 210 with respect to a region of interest 120, in accordance with some aspects of the present disclosure.

Fig. 8 is a schematic diagram illustrating an example of a second orientation parameter $\beta$ that defines an orientation of an imaging system 210 with respect to a region of interest 120, in accordance with some aspects of the present disclosure.

Fig. 9 is an example of an overlay image 170 including a graphical representation of a performance metric 160, in accordance with some aspects of the present disclosure.

Fig. 10 is an example of contrast agent projection data 150 representing X-ray attenuation in a region of interest 120 that includes an identified vessel 180, in accordance with some aspects of the present disclosure.

Fig. 11 is an example of contrast agent projection data 150 in which data corresponding to vessels other than an identified vessel 180 has been removed, in accordance with some aspects of the present disclosure.

Fig. 12 is a schematic diagram illustrating an example of the operations of orienting a 3D vascular model 190 with respect to a virtual X-ray source $230_V$ and a virtual X-ray detector $240_V$, and adjusting an estimated contrast agent distribution C(x,y,z) in the 3D vascular model 190, in accordance with some aspects of the present disclosure.

DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a method of providing projection data. The projection data is provided using spectral CT data and spectral X-ray projection data that both represent a region of interest. In some examples, the region of interest is a heart. However, it is to be appreciated that these serve only as examples, and that the region of interest may alternatively be another anatomical region, such as a leg, an arm, a brain, and so forth. Thus, the spectral CT data, the spectral X-ray projection data, and consequently the projection data, may in general represent any anatomical region.

In some examples, the projection data that is provided by the method is subsequently analyzed in order to provide a value of a blood flow parameter for one or more vessels. In some examples, the vessel is a coronary artery in the region of interest. However, it is to be appreciated that the vessel may in general be any artery, vein, or ventricle, in the anatomy. It is also to be appreciated that the determination of a value of a blood flow parameter for multiple vessels may include the determining of individual values of a blood flow parameter for multiple vessels, or the determining of a single value of a blood flow parameter for multiple vessels.

In some examples, the blood flow parameter that is provided by the method is a fractional flow reserve "FFR". However, it is to be appreciated that this serves only as an example, and that the method disclosed herein may alternatively be used to calculate values for other blood flow parameters, such as, and without limitation, a blood flow velocity, a blood pressure, a blood flow transit time, a volumetric blood flow value, an iFR value, a CFR value, a TIMI flow grade, an IMR value, and an HMR value, a hyperemic stenosis resistance "HSR" value, a zero flow pressure "ZFP" value, and an instantaneous hyperemic diastolic velocity-pressure slope "IHDVPS" value.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD

As mentioned above, there is a need for improvements in the provision of projection data by spectral X-ray projection imaging systems.

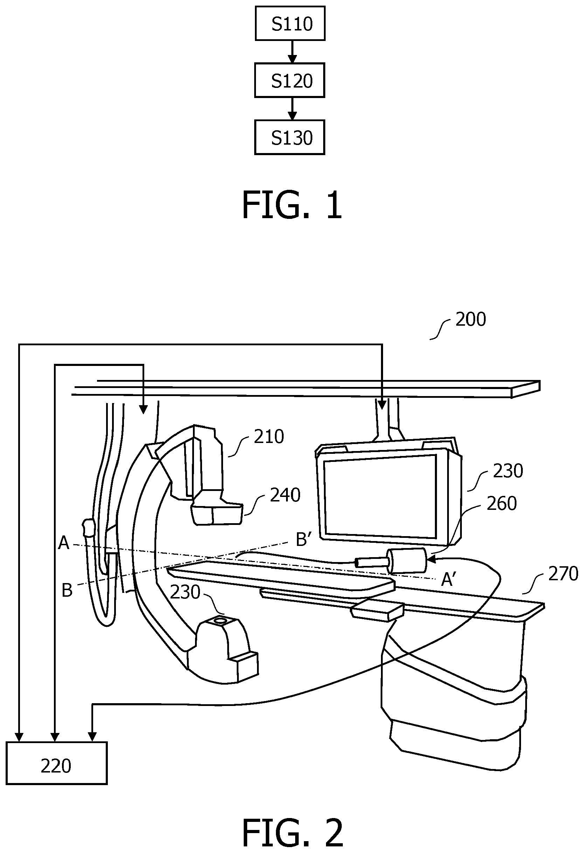

Fig. 1 is a flowchart illustrating an example of a computer-implemented method of providing projection data, in accordance with some aspects of the present disclosure. Fig. 2 is a schematic diagram illustrating an example of a system 200 for providing projection data, in accordance with some aspects of the present disclosure. Operations described in relation to the method illustrated in Fig. 1, may also be performed by the system 200 illustrated in Fig. 2. Likewise, operations described in relation to the system 200 may also be performed in the method described with reference to Fig. 1. With reference to Fig. 1, the computer-implemented method of providing projection data includes:

> receiving S110 spectral computed tomography, CT, data 110 representing a region of interest 120, the spectral CT data defining X-ray attenuation within the region of interest in a plurality of different energy intervals $\Delta E_{1..m}$;
> receiving S120 spectral X-ray projection data 130 representing the region of interest 120, the spectral X-ray projection data defining X-ray attenuation within the region of interest in the plurality of energy intervals $\Delta E_{1..m}$, and being generated using a set value of one or more imaging parameters 140; and
> extracting S130, from the spectral X-ray projection data 130, material projection data 150 representing X-ray attenuation of one or more materials in the region of interest 120, to provide the projection data; and
> wherein the extracting S130 material projection data 150 from the spectral X-ray projection data 130, or the setting of the value of the one or more imaging parameters 140, is performed using the received spectral CT data 110.

In the above method, the extraction of the material projection data from the spectral X-ray projection data, or the setting of the value of the one or more imaging parameters, is performed using spectral CT data for the region of interest. The spectral CT data represents the volumetric extent of features in the region of interest, and consequently provides more accurate spatial information on the spectral attenuation by the features. By extracting the material projection data using the spectral CT data, spatially-accurate spectral information for the features is used to extract the material projection data, and consequently the resulting material projection data for the features is more specific to the intended material(s). By setting the value(s) of imaging parameters, such as e.g. a dose, a spectrum of X-rays, and an orientation of the imaging system used to generate the spectral X-ray projection data, using the spectral CT data, it can be ensured that the spectral X-ray projection data includes sufficient information to accurately extract the material projection data for a material. Consequently, both uses of the spectral CT data provide that the extracted material projection data is more specific and more accurate to the intended material(s). This enables a physician to make a more accurate diagnosis of the region of interest from the extracted material projection data.

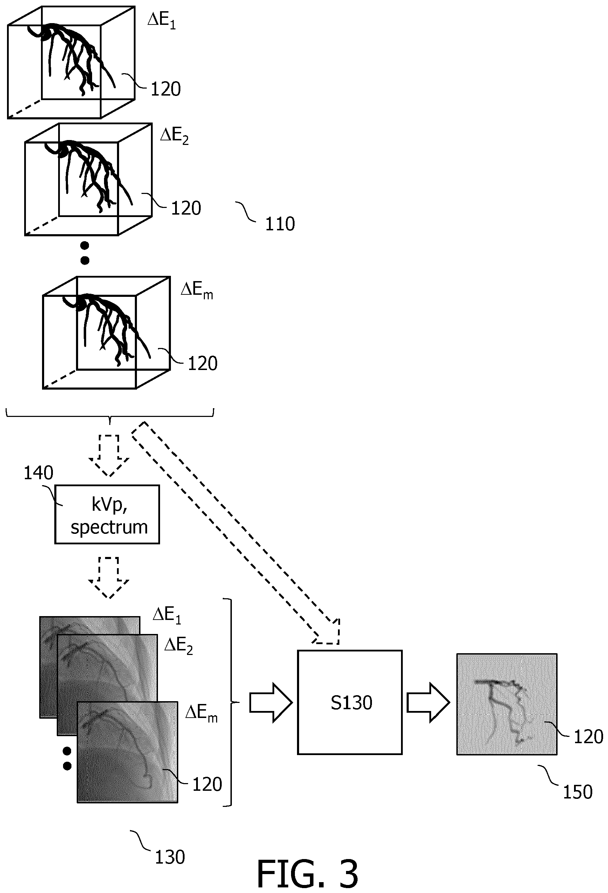

The method illustrated in Fig. 1 is also described with reference to Fig. 3, which is a schematic diagram illustrating an example of a computer-implemented method of providing projection data, in accordance with some aspects of the present disclosure.

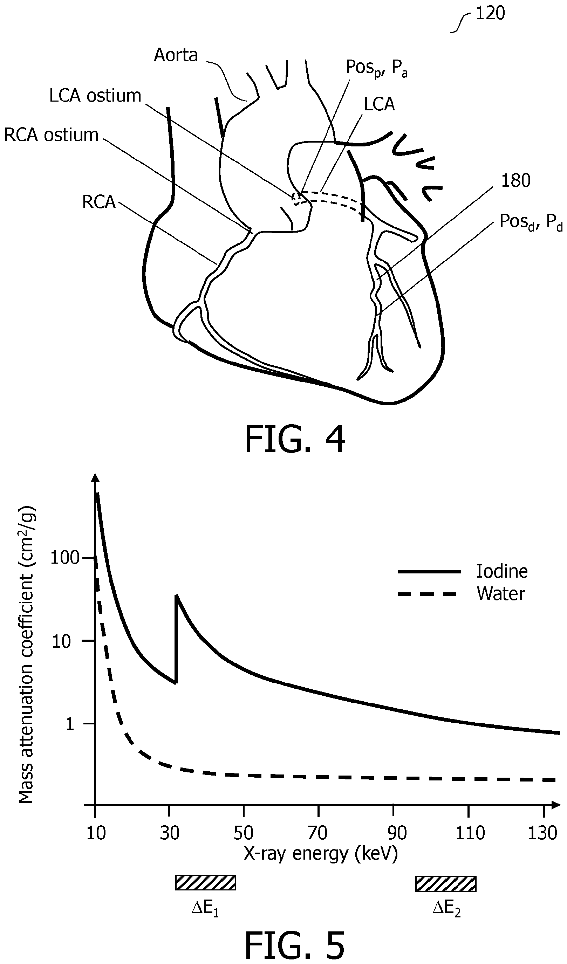

With reference to Fig. 1 - Fig. 3, in the operation S110, spectral CT data 110 is received. The spectral CT data 110 represents a region of interest 120, and defines X-ray attenuation within the region of interest in a plurality of different energy intervals $\Delta E_{1..m}$. The region of interest 120 that is represented by the spectral CT data 110 may in general be any part of the anatomy. In some examples, the spectral CT data represents the heart. Fig. 4 is a schematic diagram illustrating a heart, including an example of a vessel 180, in accordance with some aspects of the present disclosure. The heart illustrated in Fig. 4 is labelled with the left coronary artery, LCA, the right coronary artery, RCA, and the LCA ostium and RCA ostium that respectively define the openings of these arteries in the aorta. The spectral CT data 110 that is received in the operation S110 may for instance represent the heart illustrated in Fig. 4. In some examples that are described later, a value of a blood flow parameter is determined from projection data that is acquired for the heart. The value of the blood flow parameter may for example be determined for the vessel 180 of the heart illustrated in Fig. 4, i.e. the left coronary artery.

The spectral CT data 110 that is received in the operation S110 may in general be raw data, i.e. data that has not been reconstructed into volumetric image, or it may be image data, i.e. data that represents a reconstructed volumetric image. The spectral CT data 110 may be received by the one or more processors 220 illustrated in Fig. 2. The spectral CT data 110 may be received via any form of data communication, including wired, optical, and wireless communication. By way of some examples, when wired or optical communication is used, the communication may take place via signals transmitted on an electrical or optical cable, and when wireless communication is used, the communication may take place via RF or optical signals. In general, the one or more processors 220 may receive the spectral CT data 110 from an imaging system, such as a spectral CT imaging system, or from another source, such as a computer readable storage medium, the Internet, or the Cloud, for example.

With continued reference to Fig. 1, the spectral CT data 110 that is received in the operation S110 may in some examples be angiographic data. In these examples, the angiographic spectral CT data represents an injected contrast agent within a vascular region of interest. As described above with reference to Fig. 4, the angiographic spectral CT data may for instance represent an injected contrast agent in a heart. In this regard, the angiographic spectral CT data may be generated subsequent to the injection of a contrast agent into the vasculature. The contrast agent may include a substance such as iodine, or a Lanthanide such as gadolinium, or another substance that provides visibility of a blood flow into which the contrast agent is injected.

With continued reference to Fig. 1, the spectral CT data 110 that is received in the operation S110 may be generated by a spectral CT imaging system. A spectral CT imaging system generates spectral CT data whilst rotating, or stepping, an X-ray source-detector arrangement around an imaging region. Examples of spectral CT imaging systems include cone beam spectral CT imaging systems, photon counting spectral CT imaging systems, dark-field spectral CT imaging systems, and phase contrast spectral CT imaging systems. By way of an example, the spectral CT data 110 may be generated by the Spectral CT 7500 that is marketed by Philips Healthcare, Best, The Netherlands.

Instead of being generated by a spectral CT imaging system, the spectral CT data 110 that is received in the operation S110 may alternatively be generated by a spectral X-ray projection imaging system. Spectral X-ray projection imaging systems typically include a support arm such as a so-called "C-arm", also known as a gantry, that supports an X-ray source and an X-ray detector. Spectral X-ray projection imaging systems may alternatively include a support arm with a different shape to this example, such as an O-arm, for example. Spectral X-ray projection imaging systems typically generate projection data with the support arm held in a static position with respect to an imaging region during the acquisition of projection data. The projection data may be used to generate a 2D image. However, spectral X-ray projection imaging systems may also acquire spectral CT data, i.e. data that can be reconstructed into a volumetric image. A spectral X-ray projection imaging system may acquire spectral CT data for a region of interest by rotating its support arm around an axis of rotation and acquiring spectral X-ray projection data from multiple rotational angles around the axis of rotation. The set of spectral X-ray projection data from the multiple rotational angles represents spectral CT data because this may be reconstructed to provide a volumetric image. Various image reconstruction techniques may be used to reconstruct the set of spectral X-ray projection data from the multiple rotational angles into a volumetric image. These techniques process the spectral CT data in a similar manner as spectral CT data that is generated by a spectral CT imaging system. Thus, the spectral CT data 110 that is received in the operation S110 may alternatively be generated by a spectral X-ray projection imaging system.

An example of a spectral X-ray projection imaging system 210 is illustrated in Fig. 2. The spectral X-ray projection imaging system 210 illustrated in Fig. 2 includes an X-ray source 230 and an X-ray detector 240. The X-ray source 230 and the X-ray detector 240 are mechanically coupled to a support arm, and which in the illustrated example is in the form of a C-arm. In-use, the X-ray source 230 and X-ray detector 240 are positioned by means of the support arm in order to provide a desired orientation of the imaging system 210 with respect to a region of interest. When the desired orientation is reached, the spectral X-ray projection imaging system 210 generates the spectral X-ray projection data 130 with its support arm held static in this orientation with respect to the region of interest. As mentioned above, the spectral X-ray projection imaging system 210 may generate spectral computed tomography, CT, data 110 by rotating its support arm around an axis of rotation and acquiring spectral X-ray projection data from multiple rotational angles around the axis of rotation. Examples of this axis of rotation are illustrated via the dashed lines A- A' and B - B' in Fig. 2.

The spectral CT data 110 that is received in the operation S110 defines X-ray attenuation within the region of interest in a plurality of different energy intervals $\Delta E_{1..m}$. In general there may be two or more energy intervals; i.e. $m$ is an integer, and $m \geq 2$. The ability to generate data that defines X-ray attenuation data in multiple different energy intervals $\Delta E_{1..m}$ distinguishes a spectral CT imaging system from a conventional CT imaging system. By processing the data from the multiple different energy intervals, a distinction can be made between media that have similar X-ray attenuation values when measured within a single energy interval, and which would be indistinguishable from attenuation data generated by a conventional CT imaging system. In this regard, various different configurations of X-ray source and X-ray detector may be used in a spectral CT imaging system to generate the spectral CT data 110 that is received in the operation S110, some of which are now described with reference to the spectral X-ray projection imaging system 210 illustrated in Fig. 2.

In general, a spectral X-ray projection imaging system includes an X-ray source 230 and an X-ray detector 240. Together, the X-ray source 230 and the X-ray detector 240 generate X-ray attenuation data in a plurality of different energy interval $\Delta E_{1..m}$. The X-ray source 230 illustrated in Fig. 2 may in general be provided by multiple monochromatic sources, or by one or more polychromatic sources, and the X-ray detector 230 may be provided by: a common detector for detecting multiple different X-ray energy intervals, or multiple detectors wherein each detector detects a different X-ray energy interval $\Delta E_{1..m}$, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

With reference to Fig. 2, in general, a discrimination between attenuation in different X-ray energy intervals $\Delta E_{1..m}$ may be provided at the X-ray source by temporally switching the tube voltage of a single X-ray source 230, i.e. by "rapid kVp switching", or by temporally switching, or filtering, the emission of X-rays from multiple X-ray sources 230. In such configurations, a common X-ray detector 240 may be used to detect X-rays across multiple different energy intervals, X-ray attenuation data for each of the energy intervals $\Delta E_{1..m}$ being generated in a time-sequential manner. Alternatively, a discrimination between attenuation in different X-ray energy intervals $\Delta E_{1..m}$ may be provided at the X-ray detector 240 by using a multi-layer detector, or a photon counting detector. Such detectors can detect X-rays from multiple X-ray energy intervals $\Delta E_{1..m}$ near-simultaneously, and thus there is no need to perform temporal switching at the X-ray source 230. Thus, a multi-layer X-ray detector 240, or a photon counting X-ray detector 240, may be used in combination with a polychromatic X-ray source 230 to generate X-ray attenuation data in a plurality of different energy intervals $\Delta E_{1..m}$.

Other configurations of the aforementioned X-ray sources 230 and X-ray detectors 240 may alternatively be used to provide X-ray attenuation data in a plurality of different energy intervals $\Delta E_{1..m}$. For example, in a yet further configuration, the need to sequentially switch different X-ray sources 230 emitting X-rays in different energy intervals may be obviated by mounting X-ray source-detector pairs to a support arm, or gantry at rotationally-offset positions around an axis of rotation. In this configuration, each source-detector pair operates independently, and a separation between the X-ray attenuation data in the different energy intervals $\Delta E_{1..m}$ is facilitated by virtue of the rotational offsets of the source-detector pairs. Improved separation between the X-ray attenuation data for the different energy intervals $\Delta E_{1..m}$ may be achieved with this configuration by applying an energy-selective filter to the X-ray detector(s) 240 in order to reduce the effects of X-ray scatter.

In yet another configuration, X-ray attenuation data in a plurality of different energy intervals $\Delta E_{1..m}$ may be provided by intercepting the X-ray beam in a conventional X-ray imaging system with one or more spectral filters. For example, a single filter may be mechanically moved into, and out of, the X-ray beam generated by a polychromatic X-ray source 230 in order to temporally control the spectrum of X-rays detected by an X-ray detector 240. The filter may for instance transmit only a portion of the spectrum of X-rays emitted by the polychromatic X-ray source 230, and thereby provide X-ray attenuation data for a first energy interval when the filter intercepts the beam. When the filter is removed from the beam, X-ray attenuation data is provided for a second energy interval, e.g. for the complete spectrum emitted by the polychromatic X-ray source. In so doing, X-ray attenuation data for the different energy intervals $\Delta E_{1..m}$ is generated in a time-sequential manner. Multiple filters, each with a different X-ray transmission spectrum, may be sequentially switched into, and out of, the X-ray beam in order to increase the number of energy intervals.

It is noted that whilst the generation of the spectral CT data 110 that is received in the operation S110 was described above with reference to a spectral X-ray projection imaging system, the spectral CT data 110 may, as mentioned above, alternatively be generated by a spectral CT imaging system. It is also noted that similar configurations of the X-ray source 230 and the X-ray detector 240 may be used in the spectral CT imaging system to provide the spectral CT data 110.

Returning to Fig. 1, in the operation S120 spectral X-ray projection data 130 is received. The spectral X-ray projection data 130 represents the region of interest 120, and defines X-ray attenuation within the region of interest in the plurality of different energy intervals $\Delta E_{1..m}$. In general there may be two or more energy intervals; i.e. $m$ is an integer, and $m \geq 2$, as described above in relation to the spectral CT data 110. The spectral X-ray projection data 130 may be received by the one or more processors 220 illustrated in Fig. 2. The spectral X-ray projection data 130 may be received via any form of data communication, as described above in relation to the spectral CT data 110. In general, the one or more processors 220 may receive the spectral X-ray projection data 130 from a spectral X-ray projection imaging system, or from another source, such as a computer readable storage medium, the Internet, or the Cloud, for example.

With continued reference to Fig. 1, the spectral X-ray projection data 130 that is received in the operation S120 may in some examples be angiographic data. In these examples, the angiographic spectral X-ray projection data represents an injected contrast agent within a vascular region of interest. Thus, the angiographic spectral X-ray projection data may be generated subsequent to the injection of a contrast agent into the vasculature, as described above in relation to the angiographic spectral CT data.

With continued reference to Fig. 1, the spectral X-ray projection data 130 that is received in the operation S120 may be generated by a spectral X-ray projection imaging system. The spectral X-ray projection data 130 may for example be generated by the spectral X-ray projection imaging system 210 described above with reference to Fig. 2. Various different configurations of the X-ray source 230 and the X-ray detector 240 may be used in the spectral X-ray projection imaging system 210, as described above. In one example, the same spectral X-ray projection imaging system 210 is used to generate both the spectral X-ray projection data 130 and the spectral CT data 110. This has the advantage of limiting the amount of motion, and also the amount of change in shape of the region of interest between acquisition of the two sets of data. This improves their correspondence by obviating the need to move a subject between imaging systems in order to acquire the two sets of data.

With continued reference to Fig. 1, in the operation S130, material projection data 150 representing X-ray attenuation of one or more materials in the region of interest 120, is extracted from the spectral X-ray projection data 130 to provide projection data for the one or more materials.

In general, the spectral X-ray projection data 130 that is received in the operation S110 represents the X-ray attenuation arising from multiple materials in the region of interest. The goal of the extracting operation S130 is to extract the material projection data 150 for one or more desired materials from the spectral X-ray attenuation data. In the examples described below, material projection data 150 is extracted for an injected contrast agent that includes iodine. However, it is to be appreciated that material projection data 150 may alternatively, or additionally, be extracted for other types of materials, such as for example fat, bone, water, soft tissue, vessel calcification, metal, and air.

Various material decomposition algorithms may be used to extract the material projection data 150 in the operation S130. One example of a material decomposition algorithm that may be used in the operation S130 is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another suitable material decomposition algorithm is disclosed in a document by Roessl, E. and Proksa, R., "K-edge imaging in X-ray computed tomography using multi-bin photon counting detectors", Phys Med Biol. 2007 Aug 7, 52(15):4679-96. Another suitable material decomposition algorithm is disclosed in the published PCT patent application WO/2007/034359 A2. Another suitable material decomposition algorithm is disclosed in a document by Silva, A. C., et al., "Dual-energy (spectral) CT: applications in abdominal imaging", RadioGraphics 2011; 31(4):1031-1046.

In general, the X-ray attenuation spectrum of a material includes a contribution from Compton Scatter and a contribution from the Photo-electric effect. The attenuation due to Compton scatter is relatively similar for different materials, whereas the attenuation from the Photo-electric effect is strongly material-dependent. Both Compton Scatter and the Photo-electric effect exhibit an energy dependence, and it is this effect that is exploited by material decomposition algorithms to analyze spectral X-ray projection data in order to distinguish between different materials.

In general, material decomposition algorithms operate by decomposing the attenuation spectrum of an absorbing medium into contributions from an assumed set of "basis" materials. The energy-dependent X-ray attenuation of the assumed basis materials is typically modelled as a combination of absorption from Compton Scatter, and the Photo-electric effect. Some materials also have a k-absorption edge "k-edge" energy that is within the energy range used by diagnostic X-ray imaging systems, and this effect may also be exploited in order to distinguish between different materials. The spectral decomposition algorithm then seeks to estimate an amount of each of the basis materials that is required to produce the measured amount of X-ray attenuation at the two or more energy intervals. Water and iodine are examples of basis materials that are often separated in clinical practice using a so-called two-material decomposition algorithm. Non-fat soft tissue, fat, and iodine, are examples of basis materials that are often separated in clinical practice using a three-material decomposition algorithm.

As mentioned above, if the k-absorption edge "k-edge" energy of any of the basis materials is within the energy range used by diagnostic X-ray imaging systems, i.e., approximately 30 - 120 keV, this can help to identify the contribution of a basis material. The k-edge energy for a material is defined as the minimum energy required for the Photo-electric event to occur with a k-shell electron. The k-edge occurs at a characteristic energy for each material. The k-edge energy for a material is marked by a sharp increase in its X-ray attenuation spectrum at X-ray energies corresponding to the k-edge energy value. The k-edge energies of many materials that are present in the human body are too low to be detected in a diagnostic X-ray imaging system. For example, the k-edge energies of hydrogen, carbon, oxygen, and nitrogen are at energies that are less than 1 keV. However, materials such as iodine (k-edge = 33.2 keV), gadolinium (50.2 keV), gold (80.7 keV), platinum (78.4 keV), tantalum (67.4 keV), holmium (55.6 keV), and molybdenum (k-edge = 20.0 keV) have k-edge energy values that permit their distinction in spectral X-ray attenuation data.

By way of an example, Fig. 5 is a graph illustrating the dependence of the mass attenuation coefficient with X-ray energy for two example materials, iodine and water. The mass attenuation coefficient represented in Fig. 5 represents the X-ray attenuation. The sharp increase in X-ray attenuation for iodine at 33.2 keV facilitates a separation between the contributions of each of these materials to a combined attenuation spectrum. In this example, iodine and water can be separated by providing one energy interval $\Delta E_1$ close to the k-edge energy of 33.2 keV, and another energy interval $\Delta E_2$ significantly above the k-edge energy. Thus, the operation S130 may involve the use of a material decomposition algorithm to extract material projection data 150 for an iodine-based contrast agent from spectral X-ray projection data 130 that includes both iodine and water.

With reference to the method illustrated in Fig. 1 and Fig. 3, the method encompasses two alternative uses of the spectral CT data 110 that is received in the operation S110. In Fig. 3 these options are illustrated by way of the dashed arrows. In a first option the left-hand arrows in Fig. 3 are followed. In this option, the value of the one or more imaging parameters (140) that are used to generate the spectral X-ray projection data (130), are set using the received spectral CT data (110). In a second option, the right-hand arrow in Fig. 3 is followed. In this option, the operation of extracting S130 material projection data 150 from the spectral X-ray projection data 130, is performed using the spectral CT data 110 that is received in the operation S110. These options are now described in more detail.

In the first option, the operation of setting of the value of one or more imaging parameters 140 for generating the spectral X-ray projection data 130, is performed using the spectral CT data 110 that is received in the operation S110. The inventors have observed that by setting of the value of the one or more imaging parameters 140 using the spectral CT data 110 in accordance with these examples, it can be ensured that the resulting material projection data is more specific to an intended material. In turn, this improvement to the material projection data facilitates a physician to make a more accurate diagnosis of the region of interest.

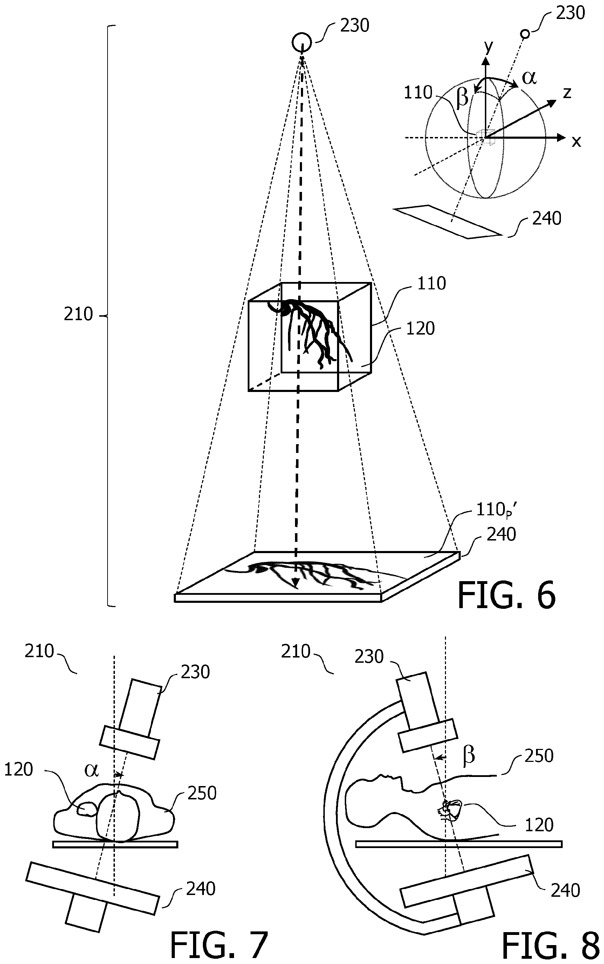

Examples relating to the first option are also with reference to Fig. 6 - Fig. 9. In these examples, the setting of the value of the one or more imaging parameters 140 may include controlling a spectrum of X-rays emitted by an X-ray source 230 of an imaging system 210 used to generate the spectral X-ray projection data 130 and/or controlling a dose of X-ray radiation emitted by the X-ray source 230 of the imaging system 210 and/or controlling an orientation of the imaging system 210 with respect to the region of interest 120.

In these examples, the setting of the value of the one or more imaging parameters 140 using the received spectral CT data 110 includes:

projecting the received spectral CT data 110 to provide simulated spectral X-ray projection data $110_P$', the received spectral CT data 110 being projected using an estimated orientation of an imaging system 210 used to generate the spectral X-ray projection data 130 with respect to the region of interest 120;
calculating, from the simulated spectral X-ray projection data $110_P$', spatially dependent values of a performance metric 160; and
setting the value of the one or more imaging parameters 140 such that the spatially dependent values of the performance metric 160 meet a predetermined criterion.

These examples are described initially with reference to Fig. 6, which is a schematic diagram illustrating an example of the operation of projecting spectral CT data 110 to provide simulated spectral X-ray projection data $110_P$', in accordance with some aspects of the present disclosure. In Fig. 6, the spectral CT data 110 is illustrated in the form of a volumetric image that is arranged between the X-ray source 230 and the X-ray detector 240 of a projection X-ray imaging system 210. The projection X-ray imaging system 210 has the same geometry as the imaging system that is used to generate the spectral X-ray projection data 130. For instance, the projection X-ray imaging system 210 has the same size of X-ray detector, and the same separation between the X-ray source 230 and the X-ray detector 240, as used in the imaging system that generates the spectral X-ray projection data 130. The dashed lines connecting the X-ray source 230 to the X-ray detector 240 illustrate the extent of the imaging beam within which X-ray attenuation data is generated by the imaging system 210. The spectral CT data 110 is projected mathematically using a ray tracing technique in which the line integral of the attenuation of virtual X-rays passing through the spectral CT data is determined for multiple paths between the X-ray source 230 and the X-ray detector 240. The line integral is determined for paths that originate at the X-ray source 230, and terminate at different positions on the X-ray radiation-sensitive surface of the X-ray detector 240. For each position on the X-ray radiation-sensitive surface of the X-ray detector 240, an integrated attenuation value is provided that represents the total amount of attenuation along the path. The integrated attenuation values are multiplied by an intensity value for the X-ray radiation emitted by the X-ray source 230 at each of the energy intervals $\Delta E_{1..m}$. The result of the multiplication may be referred to as simulated spectral X-ray projection data $110_P$' for the region of interest 120 because it represents the projected intensities of X-ray radiation data that would be obtained by imaging the region of interest 120 with the projection X-ray imaging system 210 at its current orientation with respect to the region of interest.

The simulated spectral X-ray projection data $110_P$' is obtained from the spectral CT data using an estimated orientation of the imaging system 210 that is used to generate the spectral X-ray projection data 130. In some situations, the region of interest 120 may be imaged routinely using the same orientation, i.e. a known orientation. In these situations, the estimated orientation of the imaging system 210 may be estimated from the known orientation of the imaging system 210 with respect to the region of interest 120. The orientation may alternatively be estimated from a measurement of the orientation at the time of generating the spectral X-ray projection data 130. For example, a rotational encoder, or a position encoder, or a position tracking system may be used to measure the orientation of the imaging system 210 via the first and second orientation parameters $\alpha$ and $\beta$ illustrated in Fig. 7 and Fig 8. Fig. 7 is a schematic diagram illustrating an example of a first orientation parameter $\alpha$ that defines an orientation of an imaging system 210 with respect to a region of interest 120, in accordance with some aspects of the present disclosure. The first orientation parameter $\alpha$ defines a rotational angle of a central ray of the X-ray imaging system 210 around a longitudinal axis of a subject 250. Fig. 8 is a schematic diagram illustrating an example of a second orientation parameter $\beta$ that defines an orientation of an imaging system 210 with respect to a region of interest 120, in accordance with some aspects of the present disclosure. The second positioning parameter $\beta$ defines a tilt angle of a central ray of the X-ray imaging system 210 with respect to a cranial-caudal axis of the subject 250. Thus, the orientation parameters $\alpha$ and $\beta$ may be measured at the time of generating the spectral X-ray projection data 130 and subsequently used to project the spectral CT data 110 from the same orientation with respect to the region of interest.

The simulated spectral X-ray projection data $110_P$' is then used to calculate spatially dependent values of a performance metric 160. The performance metric may represent various factors. In one example, the performance metric represents a material decomposition accuracy achieved by decomposing the simulated spectral X-ray projection data $110_P$' into one or more material types using a material decomposition algorithm. The performance metric may for instance represent spatially-dependent values of a signal-to-noise ratio that is obtained by using one of the material decomposition algorithms described above to decompose the spectral X-ray projection data $110_P$' into a material such as a contrast agent. The signal-to-noise ratio may be determined by comparing the simulated signal, intensity or line integral value to the expected noise introduced by the stochastic nature of imaging with photons given a specific simulated dose level. Alternatively, the performance metric may represent a confidence that the material type is correctly identified by a material decomposition algorithm. Confidence values may be calculated by comparing the given material data from the simulation and ray casting through the CT data to the material data obtained from the material decomposition algorithm that is applied to the simulated projection data. High confidence is achieved if both material data show the same or similar values, or if the difference is below a predefined threshold and if this similarity persists independent of expected variation in the simulated projection data. E.g. from photon noise or typical inaccuracies in the imaging system. Since the calculated values of the performance metric take into account the spectrum of the X-ray source 230 that was used to generate the spectral X-ray projection data $110_P$', as well as the spatial distribution of the attenuation of the region of interest represented in the spectral CT data 110, the performance metric provides an accurate prediction of the performance that would be obtained if the current settings of the imaging system 210 were used to generate spectral X-ray projection data 130 with the estimated orientation of the imaging system 210.

The performance metric 160 may alternatively represent other factors. For example, the performance metric 160 may represent an expected amount of X-ray dose detected in one or more of the energy intervals $\Delta E_{1..m}$ of the imaging system 210 used to generate the spectral X-ray projection data 130, or it may represent a signal-to-noise ratio that is calculated from the X-ray dose and an estimated amount of noise. The amount of noise may be estimated from measurements of the X-ray detector performance.

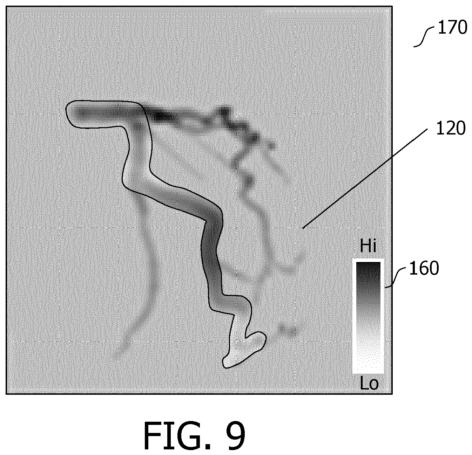

The spatially dependent values of the performance metric 160 may then be outputted. The spatially dependent values of the performance metric 160 may be outputted in various ways, including to a display device, or to a computer readable storage medium, or to a printer device, for example. In one example, the spatially dependent values of the performance metric 160 are outputted as an overlay image 170. For example, an overlay image may be outputted that includes a graphical representation of the region of interest 120 and the spatially dependent values of the performance metric 160. Fig. 9 is an example of an overlay image 170 including a graphical representation of a performance metric 160, in accordance with some aspects of the present disclosure. In Fig. 9, the overlay image 170 includes a graphical representation of the left coronary artery, and which is overlaid with the performance metric 160. The performance metric 160 may be provided graphically in various ways, including as a grayscale image as illustrated in Fig. 9 and in which dark-shaded regions represent relatively higher values for the performance metric, and light-shaded regions represent relatively lower values for the performance metric. The performance metric may alternatively be provided as e.g. a color-coded overlay image in which various colors represent the relative values of the performance metric. A user may then assess the overlay image illustrated in Fig. 9 in order to identify any parts of the region of interest in which the performance metric 160 is inadequate.

The value(s) of the one or more imaging parameters 140 are then set such that the spatially dependent values of the performance metric 160 meet a predetermined criterion. With continued reference to the example above, this operation may for example be performed by setting the value of the imaging parameter(s) such that the material decomposition accuracy, or the expected amount of X-ray dose, meets a predetermined criterion. For example, it may be specified that the material decomposition accuracy, or the X-ray dose, exceeds a threshold value in a specified portion of the region of interest. In so doing, it may be ensured that when the spectral X-ray projection data 130 is generated using the settings, the data includes sufficient information to accurately isolate a desired material from other materials that may be represented by the spectral X-ray projection data 130. Alternatively, it may be specified that the X-ray dose in a specified portion of the region of interest does not exceed another threshold value. In so doing, the amount of X-ray dose delivered to a subject may be maintained within safe limits.

As mentioned above, the imaging parameters that are set in this operation may include a spectrum of X-rays emitted by an X-ray source 230 of the imaging system 210 and/or a dose of X-ray radiation emitted by the X-ray source 230 of the imaging system 210 and/or an orientation of the imaging system 210 with respect to the region of interest 120.

The spectrum of X-rays emitted by the X-ray source 230 of the spectral X-ray projection imaging system 210 may be controlled by adjusting a tube voltage of the X-ray source 230. This technique is known as kVp switching. The spectrum of X-rays emitted by the X-ray source 230 of the spectral X-ray projection imaging system 210 may alternatively be controlled in other ways, including for example by switching on a different X-ray source 230 of the spectral X-ray projection imaging system 210, or by interrupting a beam of X-ray radiation emitted by the X-ray source 230 with a spectral filter.

The dose of X-ray radiation emitted by the X-ray source 230 of the imaging system 210 may be controlled by adjusting the duration over which the X-ray detector 240 in the spectral X-ray projection imaging system 210 is exposed to X-ray radiation from the X-ray source 230, or by interrupting a beam of X-ray radiation emitted by the X-ray source 230 with an attenuating filter that has a flat spectral response, for example.

The orientation of the imaging system 210 with respect to the region of interest 120 may be controlled by adjusting the orientation parameters $\alpha$, and $\beta$ that were described above with reference to Fig. 6. The orientation of the imaging system 210 with respect to the region of interest 120 may be controlled automatically. For instance, the one or more processors 220 may transmit control signals to one or more actuators that are mechanically coupled to the support arm of the spectral X-ray projection imaging system 210 illustrated in Fig. 2, and which in combination with position feedback from the rotational encoder, or the position encoder, or the position tracking system, described above, provide a desired orientation of the imaging system 210 with respect to the region of interest 120.

The values of the imaging parameters that are set in this operation may be determined in various ways. In one example, the values for the X-ray dose and spectrum of X-rays emitted by an X-ray source 230 are determined by calculating a change in the simulated spectral X-ray projection data $110_P'$ that would be required in order for the performance metric 160 to meet the predetermined criterion, back-projecting the change through the spectral CT data 110, and consequently calculating a change in the X-ray radiation emitted by the X-ray source in each of the energy intervals $\Delta E_{1..m}$, that would be required in order to generate the change in the simulated spectral X-ray projection data $110_P'$. In another example, values for the X-ray dose and spectrum of X-rays emitted by the X-ray source 230 are adjusted incrementally. The effect of the adjustment is calculated via the performance metric 160 after each adjustment, and the adjustment is performed repetitively until the value of the performance metric 160 meets the predetermined criterion. Rather than adjusting the values for the X-ray dose and spectrum of X-rays emitted by the X-ray source 230 incrementally, these values may be adjusted iteratively using a mathematical optimization technique that is based on the value of an error function. In this case, the error function represents the difference between the desired value of the performance metric 160, and its value for the current iteration.

The values of the orientation parameters $\alpha$, and $\beta$, that are required in order for the performance metric 160 to meet the predetermined criterion may also be determined using the simulated spectral X-ray projection data $110_P'$ that is generated by the arrangement illustrated in Fig. 6. For instance, an initial value of the performance metric 160 may be calculated using the estimated orientation of the imaging system 210 that is estimated in the manner described above. Subsequently, the effect on the performance metric 160 of incremental adjustments to the orientation parameters $\alpha$,

and β may be calculated, and used to determine the values of the orientation parameters for which the performance metric 160 meets the predetermined criterion. Alternatively, a mathematical optimization technique may be used to iteratively adjust the values of the orientation parameters α, and β, in response to the value of an error function. The error function represents the difference between the desired value of the performance metric 160, and its value for the current iteration. The effect of each adjustment is calculated via the performance metric 160 after each adjustment. The value of the error function is likewise used to adjust the values of the orientation parameters repetitively until the value of the performance metric 160 meets a predetermined criterion.

Thus, by using the received spectral CT data 110 to set the value of the one or more imaging parameters 140 in accordance with this first option, it may be ensured that the extracted material projection data 150 includes sufficient information to accurately represent the desired material.

The second option, and in which the operation of extracting S130 material projection data 150 from the spectral X-ray projection data 130, is performed using the spectral CT data 110 that is received in the operation S110, is now described. In examples in accordance with this second option, the method described with reference to Fig. 1 includes:

projecting the received spectral CT data 110 to provide simulated spectral X-ray projection data $110_P$', the received spectral CT data 110 being projected using an estimated orientation of an imaging system 210 used to generate the spectral X-ray projection data 130 with respect to the region of interest 120; and

the extracting S130 material projection data 150 from the spectral X-ray projection data 130, comprises subtracting the simulated spectral X-ray projection data $110_P$' from the spectral X-ray projection data 130 to provide subtraction data representing the region of interest 120.

In these examples, the operation of projecting of the received spectral CT data 110 is performed using the same techniques described above with reference to Fig. 6 - Fig. 8. In some examples, the projecting of the received spectral CT data 110 may be performed with a subset of the spectral CT data 110. The subset of the spectral CT data 110 may represent one or more background materials other than material(s) represented by the material projection data (150). This results in simulated spectral X-ray projection data $110_P$' for the background material(s). Consequently, the subtraction that is performed in the extracting operation S130 removes the attenuation arising from these background materials, and the resulting subtraction data represents the desired material without the background material(s). Since the subtraction is performed using projection data that is obtained by projecting the spectral CT data 110, i.e. volumetric data, the subtracted data has a spectrally-accurate spatial distribution of the attenuation of the desired material.

In these examples, the method described with reference to Fig. 1 may also include:

generating, from the spectral CT data 110, background material data defining X-ray attenuation of one or more materials represented in the spectral CT data other than the one or more materials represented by the material projection data 150; and

the projecting the received spectral CT data 110, is performed with the background material data to provide simulated background material projection data; and

the extracting S130 material projection data 150 from the spectral X-ray projection data 130 comprises subtracting the simulated background material projection data from the spectral X-ray projection data to provide the subtraction data.

In these examples, the operation of generating the background material data from the spectral CT data 110 may be performed by applying a material decomposition algorithm to the spectral CT data 110, the material decomposition algorithm being configured to identify X-ray attenuation arising from the one or more materials represented in the spectral CT data 110 other than the one or more materials represented by the material projection data 150. Any of the material decomposition algorithms described above may be used for this purpose.

An imaging scenario is now described in order to illustrate the benefits of this example. In this scenario, the region of interest is the heart, and material projection data is desired for the contrast agent in a coronary artery in order to determine a value of a blood flow parameter. In this scenario, spectral X-ray projection data 130 for the heart typically also represents bony structures such as the ribs, and the spine. These bony structures unavoidably overlap with the heart during imaging. In this scenario, it would be advantageous to accurately remove the effects of attenuation by the bony structures from the spectral X-ray projection data 130 in order to determine a value of the blood flow parameter. By generating background material data for bone, projecting the background material data for bone to provide simulated bone projection data, and extracting the contrast agent projection data 150 from the spectral X-ray projection data 130 by subtracting the simulated bone projection data from the spectral X-ray projection data, subtraction data is provided for the contrast agent without the bone. In these examples, the material projection data 150 is provided by the subtraction data. The subtraction data provides an improved visualization of the region of interest for the contrast agent because the bone is removed, and this also facilitates a more accurate calculation of the blood flow parameter.

It is noted that in these examples, the operation of extracting S130 the material projection data may involve applying a material decomposition algorithm to the subtraction data. This results in the isolation of the material projection data for the desired material from other materials that may also be present in the subtraction data, the effects of which were not removed in the subtraction operation.

The operation of subtracting the simulated background material projection data from the spectral X-ray projection data 130 in these examples may be performed in various ways.

In one example, the operation of subtracting the simulated background material projection data from the spectral X-ray projection data 130 comprises:

subtracting image intensity values in each of the plurality of energy intervals $\Delta E_{1..m}$ in the simulated background material projection data from image intensity values in corresponding energy intervals $\Delta E_{1..m}$ at corresponding positions in the region of interest 120 in the spectral X-ray projection data 130.

As described above, the energy intervals $\Delta E_{1..m}$ may be defined by different layers of a multi-layer detector, or they may be defined by the tube voltage of the X-ray source. In this example, for each detector layer, or for each tube voltage that is used to generate the spectral X-ray projection data 130, simulated background material projection data is generated from the spectral CT data 110. The simulated background material projection data is subtracted from the spectral X-ray projection data 130 at corresponding energy intervals $\Delta E_{1..m}$ to provide the subtraction data for each of the energy intervals $\Delta E_{1..m}$. In so doing, the resulting subtraction data is spectrally accurate.

In another example, the operation of subtracting simulated background material projection data from the spectral X-ray projection data 130 comprises:

subtracting image intensity values within an energy range selected from the plurality of energy intervals $\Delta E_{1..m}$ in the simulated background material projection data, from image intensity values within the same energy range and in corresponding positions in the region of interest 120 in the spectral X-ray projection data 130.

An energy range may for defined by selecting energy intervals from the energy intervals $\Delta E_{1..m}$. In this case, the subtraction is performed for corresponding ranges of energy intervals. In so doing, the resulting subtraction data is also spectrally accurate.

Further examples that relate to the second option, are now described in which projection data for vessels other than a vessel of interest is removed from the subtraction data.

The inventors have observed that despite the extracted material projection data relating to a specific material, e.g. a contrast agent, the extracted material projection data is susceptible to confounding effects that arise from the projected nature of this data. For example, overlapping vessels in the extracted material projection data that include the same contrast agent material give rise to overlap regions in the projection data in which the attenuation from the overlapping vessels is combined. This can be confusing to a reviewing physician. It can also give rise to errors in calculations of blood flow parameters from the extracted material projection data because such calculations are typically rely upon accurate values for the contrast agent intensity along a vessel. In the following examples, data for vessels other than a vessel of interest is removed from the subtraction data, which provides improved visualization of an injected contrast agent in a vessel of interest, and also facilitates the calculation of more accurate blood flow parameters for the vessel of interest.

In these examples, the material projection data 150 represents X-ray attenuation of an injected contrast agent in a plurality of vessels in the region of interest 120, and the method described with reference to Fig. 1 also includes:

receiving input identifying at least one of the vessels 180 in the region of interest 120; and
removing, from the subtraction data, data corresponding to one or more vessels other than the identified at least one of the vessels 180.

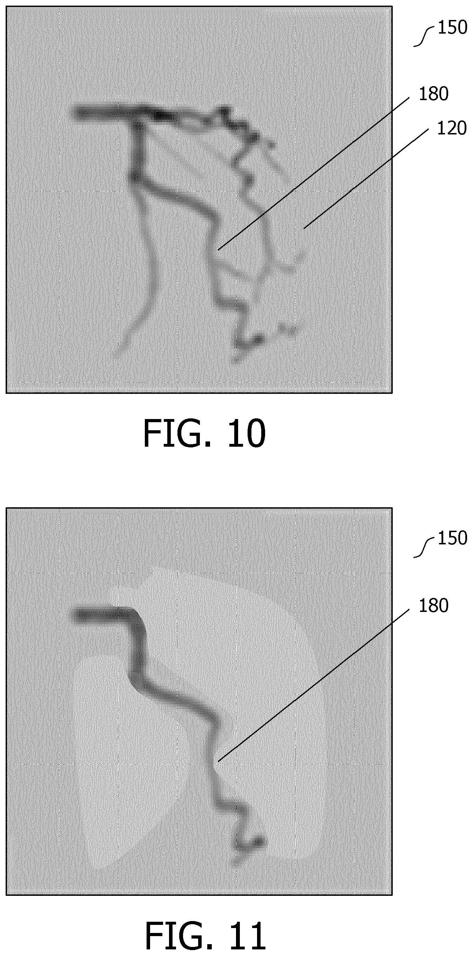

These examples are described with reference to Fig. 10 - Fig. 12. Fig. 10 is an example of contrast agent projection data 150 representing X-ray attenuation in a region of interest 120 that includes an identified vessel 180, in accordance with some aspects of the present disclosure. With reference to Fig. 10, projection data is desired for the identified vessel 180. Projection data may be desired for this vessel in order to determine a value of a blood flow parameter for the vessel without the confounding effects of overlapping data from neighboring vessels. Fig. 10 depicts the projection data prior to the removal of data for vessels other than the identified vessel 180. Fig. 11 is an example of contrast agent projection data 150 in which data corresponding to vessels other than an identified vessel 180 has been removed, in accordance with some aspects of the present disclosure. As may be appreciated from Fig. 11, by removing data for the other vessels, visibility of the identified vessel 180 is improved because the attenuation arising from other vessels, and which may overlap with the vessel 180, has been removed. The data for the identified vessel 180 may then be used to accurately determine a value of a blood flow parameter for the identified vessel 180, without the confounding effects of data for other vessels that include the same contrast agent.

In these examples, the input identifying the vessel(s) 180 in the region of interest 120 may be provided by a user, or it may be provided automatically. User input identifying the vessel(s) 180 may be received from a user input device operating in combination with a displayed image of the region of interest. The image may be generated from the spectral X-ray projection data 130, or the spectral CT data 110, for example. Input identifying the vessel(s) 180 may be provided automatically by using a feature detector, or a trained neural network, to identify the vessel(s) 180 in the spectral X-ray projection data 130, or the spectral CT data 110. A segmentation operation may also be performed on such images in order to assist in identifying the vessel. Various segmentation algorithms are known for this purpose. A combination of these approaches may also be used in which the feature detector, or the trained neural network, automatically identifies a candidate vessel, and which is then selectable by a user via a user input device. The vessel may be identified based on a detection of contrast agent in such images, or based on the presence of an anomaly, such as a stenosis in the vessel. By way of an example, a feature detector, or a trained neural network may be used to identify the left coronary artery of a cardiac image, and also a stenosis therein, and to identify the vessel as a candidate vessel.

In one example, a projection operation is used to remove the data for the one or more vessels other than the identified vessel(s) 180. In this example, the removing of data corresponding to one or more vessels other than the identified at least one of the vessels 180 comprises:

segmenting the received spectral CT data 110 to provide a 3D vascular model 190 representing the plurality of vessels in the region of interest 120;
orienting the 3D vascular model 190 with respect to a virtual X-ray source $230_v$ and a virtual X-ray detector $240_v$ representing the imaging system 210 such that a simulated projection of the 3D vascular model obtained by projecting virtual X-rays from the virtual source $230_v$ onto the virtual detector $240_v$ through the 3D vascular model 190 provides simulated projection data $190_P$' in which the positions of the vessels correspond to the positions of the vessels in the spectral X-ray projection data 130;
adjusting an estimated contrast agent distribution C(x,y,z) in the 3D vascular model 190 such that the simulated projection of the estimated contrast agent distribution obtained using the oriented 3D vascular model 190 matches a distribution of the contrast agent in the contrast agent material projection data 150;
wherein the projecting the received spectral CT data 110 is performed using the oriented 3D vascular model and the estimated contrast agent distribution; and
wherein the removing data corresponding to one or more vessels other than the identified at least one of the vessels 180, from the contrast agent material projection data 150, comprises subtracting the simulated spectral X-ray projection data obtained using the oriented 3D vascular model and the estimated contrast agent distribution for the one or more vessels other than the identified at least one of the vessels 180, from the spectral X-ray projection data 130.

Since, in this example, the data for the vessel(s) other than the identified vessel(s) 180, is removed using an estimated contrast agent distribution C(x,y,x) that, when projected, gives simulated projection data that matches the distribution of the contrast agent in the contrast agent material projection data 150, the volumetric distribution of the spectral attenuation of the contrast agent in the vessel(s) other than the identified vessel(s) 180 is accurately accounted-for. The result of the subtraction operation is to provide projection data that accurately removes data for the vessel(s) other than the identified vessel(s) 180.

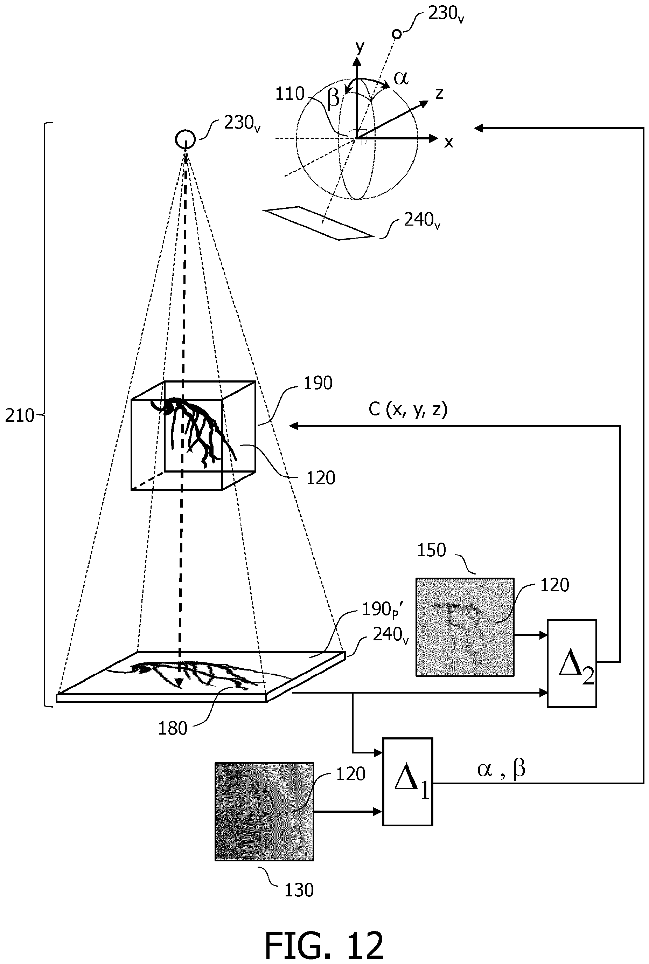

This example is described with reference to Fig. 12, which is a schematic diagram illustrating an example of the operations of orienting a 3D vascular model 190 with respect to a virtual X-ray source $230_v$ and a virtual X-ray detector $240_v$, and adjusting an estimated contrast agent distribution C(x,y,z) in the 3D vascular model 190, in accordance with some aspects of the present disclosure.

In this example, the segmenting operation may be performed using various known segmentation algorithms. The segmentation may also include processing the received spectral CT data 110 using a material decomposition algorithm in order to accurately distinguish between the vasculature and other media in the region of interest.

The goal of the operation of orienting the 3D vascular model 190 with respect to the virtual X-ray source $230_v$ and the virtual X-ray detector $240_v$, is to provide the virtual X-ray source $230_v$ and a virtual X-ray detector $240_v$ with the same orientation with respect to the region of interest as that used to generate the spectral X-ray projection data 130. This operation may be performed using a known orientation. For example, the orientation of the imaging system that was used to generate the spectral X-ray projection data 130 with respect to the region of interest may be known by measuring the orientation parameters $\alpha$, $\beta$, at the time of generating the spectral X-ray projection data 130. The orientation parameters $\alpha$, $\beta$, are described in more detail above with reference to Fig. 6. Alternatively, the orientation may be determined using an iterative approach. This latter approach is illustrated in Fig. 12, and wherein a value of a first loss function $\Delta_1$ is calculated and used to iteratively adjust the orientation parameters $\alpha$, $\beta$ until a difference between the simulated projection data $190_P$' and the spectral X-ray projection data 130, indicates that a desired accuracy has been reached. In this approach, an optimization is performed wherein the value of the first loss function $\Delta_1$ is calculated. The first loss function $\Delta_1$ represents the difference between the simulated projection data $190_P$' and the spectral X-ray projection data 130. The orientation parameters $\alpha$, $\beta$ are adjusted iteratively based on the value of the first loss function $\Delta_1$ until the value of the first loss function meets a specified criterion, and at which point the desired accuracy is reached.

The value of the first loss function $\Delta_1$ may be calculated using loss functions such as the Dice coefficient or a root mean square difference of gradient images, and so forth.

The simulated projection data $190_P$' that is used to determine the orientation is generated in the same manner as described above with reference to Fig. 6. In other words, the 3D vascular model 190 is projected mathematically using a ray tracing technique in which the line integral of the attenuation of virtual X-rays passing through the 3D vascular model 190 is determined for multiple paths between the virtual X-ray source $230_v$ and the virtual X-ray detector $240_v$.

An estimated contrast agent distribution C(x,y,z) in the oriented 3D vascular model 190 is then adjusted such that the simulated projection of the estimated contrast agent distribution obtained using the oriented 3D vascular model 190 matches a distribution of the contrast agent in the contrast agent material projection data 150. With reference to Fig. 12, this operation may be performed using an iterative approach wherein the contrast agent distribution C(x,y,z) is adjusted based on the value of a second loss function $\Delta_2$ until a difference between a simulated projection of the estimated contrast agent distribution obtained using the oriented 3D vascular model 190 matches a distribution of the contrast agent in the contrast agent material projection data 150. The contrast agent distribution C(x,y,z) is adjusted iteratively based on the value of the second loss function $\Delta_2$ until the value of the second loss function meets a specified criterion, and at which point the desired accuracy is reached. The simulated projection is generated in the same way as described above for the simulated projection data $190_P$', and with the difference that in this case the projection is performed using the estimated contrast agent distribution in the oriented 3D vascular model 190. The value of the second loss function $\Delta_2$ may be calculated using loss functions such as the Dice coefficient, or a root mean square difference, and so forth.

Having determined the distribution of contrast agent in the model that gives projected image data that corresponds to the contrast agent material projection data 150, the simulated spectral X-ray projection data that is obtained using the oriented 3D vascular model and the estimated contrast agent distribution for the one or more vessels other than the identified at least one of the vessels 180, is subtracted from the spectral X-ray projection data 130. This accurately removes the effects of attenuation arising from the vessels other than the identified vessel 180 in the region of interest because the subtraction takes account of the 3D distribution of the contrast agent in the other vessels.

In some examples, the method described with reference to Fig. 1 also includes a determination of a value of a blood flow parameter. In these examples, the material projection data 150 represents X-ray attenuation of an injected contrast agent in one or more vessels in the region of interest 120, and the method includes:

> analyzing the material projection data 150 to provide a value of a blood flow parameter for the one or more vessels in the region of interest 120; and
> outputting the value of the blood flow parameter.

The material projection data may be analyzed using various techniques in order to provide the value of the blood flow parameter. The blood flow parameter may for example represent various quantities, including for example a blood flow velocity, a blood pressure, the Fractional Flow Reserve "FFR", the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR". The value of the blood flow parameter may be outputted in various ways.

By way of an example, the blood flow parameter may be the Fractional Flow Reserve "FFR" for a vessel. With reference to Fig. 4, the FFR value may be calculated for a distal position $Pos_d$ in the left coronary artery of the vessel 180 using the equation:

$$FFR = P_d/P_a \qquad\qquad\qquad \text{Equation 1}$$

In Equation 1, $P_d$ represents a distal pressure at a distal position with respect to the stenosis, and $P_a$ represents a proximal pressure with respect to the stenosis. The FFR is typically calculated with values for $P_d$ and $P_a$ that are averaged over the cardiac cycle. FFR values above 0.8 are typically regarded as clinically insignificant, and values below 0.8 are typically considered to represent increased clinical significance. With reference to Fig. 4, the FFR may be calculated using a value for the blood pressure $P_d$ at the distal position Posd in the left coronary artery, and a value for the pressure $P_a$ at a proximal position $Pos_p$ in the left coronary artery, and which in this example is taken as the ostium of the left coronary artery. The values of the pressure at these positions may be determined from the material projection data 150 using the techniques disclosed in the document WO 2018/060529 A1, and in a document by Prevrhal, S. et al., "CT Angiographic Measurement of Vascular Blood Flow Velocity by Using Projection Data", Radiology, Vol. 261: Number 3 - December 2011, pages 923 - 929, and also in the document US 2017/0105694 A1.

The values of other blood flow parameters may similarly be calculated from the material projection data 150. For example, a technique for determining blood flow velocity values from projection data is disclosed in a document by Pereira, V. et al "Quantification of Internal Carotid Artery Flow with Digital Subtraction Angiography: Validation of an Optical Flow Approach with Doppler Ultrasound", American journal of neuroradiology, 35, pages 166 - 163.

The value of the blood flow parameter may be outputted in various ways. For instance, the value may be outputted to a display device such as the monitor 230 of the system 200 illustrated in Fig. 2. Alternatively, the value of the blood flow parameter may be outputted to a computer readable storage medium, or to a printer device, for example. In some examples, the value of the blood flow parameter is outputted as a numerical value, and in other examples, the value of the blood flow parameter is outputted graphically. For example, the value of the blood flow parameter may be outputted as a color-coded icon. The value of the blood flow parameter may also be calculated at multiple positions along a length of the vessel 180 and outputted graphically. For example, an image illustrating the vessel 180 may be outputted with the values of the blood flow parameter displayed at corresponding positions along the length of the vessel as a color-coded overlay on the image. A physician may then use the provided value(s) of the blood flow parameter to perform a clinical diagnosis on a subject.

In some examples, the method described with reference to Fig. 1 also includes outputting graphical representations of the various data that is generated or processed. For instance, the method may also include outputting a graphical representation of one or more of the following: the received spectral CT data 110, the received spectral X-ray projection data 130, the extracted material projection data 150. These graphical representations may likewise be outputted to a display device such as the monitor 230 illustrated in Fig. 2, or to a computer readable storage medium, or to a printer device.

In another example, a computer program product, is provided. The computer program product comprises instructions which when executed by one or more processors, cause the one or more processors to carry out a method of providing projection data, including:

receiving S110 spectral computed tomography, CT, data 110 representing a region of interest 120, the spectral CT data defining X-ray attenuation within the region of interest in a plurality of different energy intervals $\Delta E_{1..m}$;
receiving S120 spectral X-ray projection data 130 representing the region of interest 120, the spectral X-ray projection data defining X-ray attenuation within the region of interest in the plurality of energy intervals $\Delta E_{1..m}$, and being generated using a set value of one or more imaging parameters 140; and
extracting S130, from the spectral X-ray projection data 130, material projection data 150 representing X-ray attenuation of one or more materials in the region of interest 120, to provide the projection data; and
wherein the extracting S130 material projection data 150 from the spectral X-ray projection data 130, or the setting of the value of the one or more imaging parameters 140, is performed using the received spectral CT data 110.

In another example, a system 200 for providing projection data, is provided. The system comprises one or more processors 220 configured to:

receive S110 spectral computed tomography, CT, data 110 representing a region of interest 120, the spectral CT data defining X-ray attenuation within the region of interest in a plurality of different energy intervals $\Delta E_{1..m}$;
receive S120 spectral X-ray projection data 130 representing the region of interest 120, the spectral X-ray projection data defining X-ray attenuation within the region of interest in the plurality of energy intervals $\Delta E_{1..m}$, and being generated using a set value of one or more imaging parameters 140; and
extract S130, from the spectral X-ray projection data 130, material projection data 150 representing X-ray attenuation of one or more materials in the region of interest 120, to provide the projection data; and
wherein the one or more processors are configured to extract the material projection data 150 from the spectral X-ray projection data 130, or to set the value of the one or more imaging parameters 140, using the received spectral CT data 110.

An example of the system 200 is illustrated in Fig. 2. It is noted that the system 200 may also include one or more of: a spectral X-ray projection imaging system 210 for generating the spectral X-ray projection data 130 and the spectral CT data 110, a spectral CT imaging system (not illustrated in Fig. 2) for generating the spectral CT data 110, a monitor 230 for displaying outputted data, an injector 260 for injecting a contrast agent into the vasculature represented in the region of interest, a patient bed 270, and a user input device configured to receive user input for use in the method, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A computer-implemented method of providing projection data, the method comprising:

    receiving (S110) spectral computed tomography, CT, data (110) representing a region of interest (120), the spectral CT data defining X-ray attenuation within the region of interest in a plurality of different energy intervals $(\Delta E_{1..m})$;
    receiving (S120) spectral X-ray projection data (130) representing the region of interest (120), the spectral X-ray projection data defining X-ray attenuation within the region of interest in the plurality of energy intervals $(\Delta E_{1..m})$, and being generated using a set value of one or more imaging parameters (140); and
    extracting (S130), from the spectral X-ray projection data (130), material projection data (150) representing X-ray attenuation of one or more materials in the region of interest (120), to provide the projection data; and
    wherein the extracting (S130) material projection data (150) from the spectral X-ray projection data (130), or the setting of the value of the one or more imaging parameters (140), is performed using the received spectral CT data (110).

2. The computer-implemented method according to claim 1, wherein the setting of the value of the one or more imaging parameters (140), is performed using the received spectral CT data (110), and comprises:

    projecting the received spectral CT data (110) to provide simulated spectral X-ray projection data $(110_P')$, the received spectral CT data (110) being projected using an estimated orientation of an imaging system (210) used to generate the spectral X-ray projection data (130) with respect to the region of interest (120);
    calculating, from the simulated spectral X-ray projection data $(110_P')$, spatially dependent values of a performance metric (160); and
    setting the value of the one or more imaging parameters (140) such that the spatially dependent values of the performance metric (160) meet a predetermined criterion.

3. The computer-implemented method according to claim 2, wherein the method further comprises:
    outputting, as an overlay image (170), a graphical representation of the region of interest (120) and the spatially dependent values of the performance metric (160).

4. The computer-implemented method according to claim 2 or claim 3, wherein the setting the value of the one or more imaging parameters (140) comprises: controlling a spectrum of X-rays emitted by an X-ray source (230) of the imaging system (210) and/or controlling a dose of X-ray radiation emitted by the X-ray source (230) of the imaging system (210) and/or controlling an orientation of the imaging system (210) with respect to the region of interest (120).

5. The computer-implemented method according to any one of claims 1-4, wherein the extracting (S130) material projection data (150) from the spectral X-ray projection data (130), is performed using the received spectral CT data (110); and wherein the method comprises:

    projecting the received spectral CT data (110) to provide simulated spectral X-ray projection data $(110_P')$, the received spectral CT data (110) being projected using an estimated orientation of an imaging system (210) used to generate the spectral X-ray projection data (130) with respect to the region of interest (120); and
    wherein the extracting (S130) material projection data (150) from the spectral X-ray projection data (130), comprises subtracting the simulated spectral X-ray projection data $(110_P')$ from the spectral X-ray projection data (130) to provide subtraction data representing the region of interest (120).

6. The computer-implemented method according to claim 5, wherein the method further comprises:

    generating, from the spectral CT data (110), background material data defining X-ray attenuation of one or more materials represented in the spectral CT data other than the one or more materials represented by the material projection data (150); and

wherein the projecting the received spectral CT data (110), is performed with the background material data to provide simulated background material projection data; and

wherein the extracting (S130) material projection data (150) from the spectral X-ray projection data (130) comprises subtracting the simulated background material projection data from the spectral X-ray projection data to provide the subtraction data.

7. The computer-implemented method according to claim 6, wherein the subtracting the simulated background material projection data from the spectral X-ray projection data (130) comprises:

subtracting image intensity values in each of the plurality of energy intervals ($\Delta E_{1..m}$) in the simulated background material projection data from image intensity values in corresponding energy intervals ($\Delta E_{1..m}$) at corresponding positions in the region of interest (120) in the spectral X-ray projection data (130).

8. The computer-implemented method according to claim 6, wherein the subtracting simulated background material projection data from the spectral X-ray projection data (130) comprises:

subtracting image intensity values within an energy range selected from the plurality of energy intervals ($\Delta E_{1..m}$) in the simulated background material projection data, from image intensity values within the same energy range and in corresponding positions in the region of interest (120) in the spectral X-ray projection data (130).

9. The computer-implemented method according to any one of claims 6-8, wherein the generating background material data from the spectral CT data (110) comprises:

applying a material decomposition algorithm to the spectral CT data (110), and wherein the material decomposition algorithm is configured to identify X-ray attenuation arising from the one or more materials represented in the spectral CT data other than the one or more materials represented by the spectral X-ray projection data (130).

10. The computer-implemented method according to claim 5, wherein the material projection data (150) represents X-ray attenuation of an injected contrast agent in a plurality of vessels in the region of interest (120), and wherein the method further comprises:

receiving input identifying at least one of the vessels (180) in the region of interest (120); and

removing, from the subtraction data, data corresponding to one or more vessels other than the identified at least one of the vessels (180).

11. The computer-implemented method according to claim 10, wherein the removing data corresponding to one or more vessels other than the identified at least one of the vessels (180) comprises:

segmenting the received spectral CT data (110) to provide a 3D vascular model (190) representing the plurality of vessels in the region of interest (120);

orienting the 3D vascular model (190) with respect to a virtual X-ray source ($230_v$) and a virtual X-ray detector ($240_v$) representing the imaging system (210) such that a simulated projection of the 3D vascular model obtained by projecting virtual X-rays from the virtual source ($230_v$) onto the virtual detector ($240_v$) through the 3D vascular model (190) provides simulated projection data ($190_P$') in which the positions of the vessels correspond to the positions of the vessels in the spectral X-ray projection data (130);

adjusting an estimated contrast agent distribution ($C(x,y,z)$) in the 3D vascular model (190) such that the simulated projection of the estimated contrast agent distribution obtained using the oriented 3D vascular model (190) matches a distribution of the contrast agent in the contrast agent material projection data (150);

wherein the projecting the received spectral CT data (110) is performed using the oriented 3D vascular model and the estimated contrast agent distribution; and

wherein the removing data corresponding to one or more vessels other than the identified at least one of the vessels (180), from the contrast agent material projection data (150), comprises subtracting the simulated spectral X-ray projection data obtained using the oriented 3D vascular model and the estimated contrast agent distribution for the one or more vessels other than the identified at least one of the vessels (180), from the spectral X-ray projection data (130).

12. The computer-implemented method according to any one of claims 1 - 11, wherein the material projection data (150) represents X-ray attenuation of an injected contrast agent in one or more vessels in the region of interest (120), and wherein the method further comprises:

analyzing the material projection data (150) to provide a value of a blood flow parameter for the one or more vessels in the region of interest (120); and
outputting the value of the blood flow parameter.

13. The computer-implemented method according to any one of claims 1 - 12, wherein the method further comprises outputting a graphical representation of one or more of the following:
the received spectral CT data (110), the received spectral X-ray projection data (130), the extracted material projection data (150).

14. A computer program product comprising instructions which when executed by one or more processors, cause the one or more processors to carry out the method according to any one of claims 1 - 13.

15. A system (200) for providing projection data, the system comprising one or more processors (220) configured to:

receive (S110) spectral computed tomography, CT, data (110) representing a region of interest (120), the spectral CT data defining X-ray attenuation within the region of interest in a plurality of different energy intervals ($\Delta E_{1..m}$);
receive (S120) spectral X-ray projection data (130) representing the region of interest (120), the spectral X-ray projection data defining X-ray attenuation within the region of interest in the plurality of energy intervals ($\Delta E_{1..m}$), and being generated using a set value of one or more imaging parameters (140); and
extract (S130), from the spectral X-ray projection data (130), material projection data (150) representing X-ray attenuation of one or more materials in the region of interest (120), to provide the projection data; and
wherein the one or more processors are configured to extract the material projection data (150) from the spectral X-ray projection data (130), or to set the value of the one or more imaging parameters (140), using the received spectral CT data (110).

S110

↓

S120

↓

S130

# FIG. 1

200

210

240

230

260

270

A

B'

B

230

A'

220

# FIG. 2

FIG. 3

FIG. 4

FIG. 5

FIG. 6

FIG. 7

FIG. 8

FIG. 9

FIG. 10

FIG. 11

FIG. 12

Europäisches
Patentamt

European
Patent Office

Office européen
des brevets

# EUROPEAN SEARCH REPORT

Application Number

EP 22 18 7238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/295249 A1 (SCHAEFER DIRK [DE] ET AL) 26 September 2019 (2019-09-26) * abstract * * figures 1-3 * * paragraph [0008] – paragraph [0106] * ----- | 1-3,5-15 | INV. A61B6/00 |
| X | US 2018/192977 A1 (JIN YANNAN [US] ET AL) 12 July 2018 (2018-07-12) * abstract * * figures 1-8 * * paragraph [0009] – paragraph [0050] * ----- | 1,2,4, 14,15 | |
| A | EP 3 944 819 A1 (KONINKLIJKE PHILIPS NV [NL]) 2 February 2022 (2022-02-02) * abstract * * figures 1-4 * * paragraph [0010] – paragraph [0071] * ----- | 1-15 | |

TECHNICAL FIELDS
SEARCHED      (IPC)

A61B

The present search report has been drawn up for all claims

| Place of search | Date of completion of the search | Examiner |
|---|---|---|
| Munich | 23 December 2022 | Moehrs, Sascha |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

..................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 18 7238

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2022

| Patent document cited in search report | | Publication date | Patent family member(s) | | Publication date |
|---|---|---|---|---|---|
| US 2019295249 | A1 | 26-09-2019 | CN | 109416833 A | 01-03-2019 |
| | | | EP | 3472804 A1 | 24-04-2019 |
| | | | JP | 6828061 B2 | 10-02-2021 |
| | | | JP | 2019523683 A | 29-08-2019 |
| | | | US | 2019295249 A1 | 26-09-2019 |
| | | | WO | 2017216248 A1 | 21-12-2017 |
| US 2018192977 | A1 | 12-07-2018 | CN | 110678125 A | 10-01-2020 |
| | | | EP | 3565472 A1 | 13-11-2019 |
| | | | JP | 7102416 B2 | 19-07-2022 |
| | | | JP | 2020503944 A | 06-02-2020 |
| | | | US | 2018192977 A1 | 12-07-2018 |
| | | | US | 2019336091 A1 | 07-11-2019 |
| | | | WO | 2018129243 A1 | 12-07-2018 |
| EP 3944819 | A1 | 02-02-2022 | EP | 3944819 A1 | 02-02-2022 |
| | | | WO | 2022023082 A1 | 03-02-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007034359 A2
- WO 2018060529 A1

- US 20170105694 A1

**Non-patent literature cited in the description**

- **BRENDEL, B. et al.** Empirical, projection-based basis-component decomposition method. *Medical Imaging 2009, Physics of Medical Imaging, edited by Ehsan Samei and Jiang Hsieh, Proc. of SPIE,* vol. 7258, 72583Y
- **ROESSL, E. ; PROKSA, R.** K-edge imaging in X-ray computed tomography using multi-bin photon counting detectors. *Phys Med Biol.,* 07 August 2007, vol. 52 (15), 4679-96
- **SILVA, A. C. et al.** Dual-energy (spectral) CT: applications in abdominal imaging. *RadioGraphics,* 2011, vol. 31 (4), 1031-1046

- **PREVRHAL, S. et al.** CT Angiographic Measurement of Vascular Blood Flow Velocity by Using Projection Data. *Radiology,* December 2011, vol. 261 (3), 923-929
- **PEREIRA, V. et al.** Quantification of Internal Carotid Artery Flow with Digital Subtraction Angiography: Validation of an Optical Flow Approach with Doppler Ultrasound. *American journal of neuroradiology,* vol. 35, 166-163